# EUROPÄISCHE PATENTSCHRIFT

(11) **EP 0 456 695 B1**
(45) Veröffentlichungstag und Bekanntmachung des Hinweises auf die Patenterteilung: **13.01.1993**
(21) Anmeldenummer: 90902621.3
(22) Anmeldetag: 08.02.1990
(51) Int. Cl.: C12Q 1/04, C12M 1/34, G01N 21/35

(54) **VERFAHREN ZUR SCHNELLEN DETEKTION VON MIKROORGANISMEN IN PROBEN UND VORRICHTUNG ZUR DURCHFÜHRUNG DES VERFAHRENS**
PROCESS AND DEVICE FOR RAPID DETECTION OF MICROORGANISMS IN SAMPLES
PROCEDE ET DISPOSITIF POUR LA DETECTION RAPIDE DE MICRO-ORGANISMES DANS DES ECHANTILLONS

(30) Priorität: 09.02.1989 DE 3903777
(43) Veröffentlichungstag der Anmeldung: 21.11.1991
(73) Patentinhaber: BRUKER Analytische Messtechnik GmbH, 76287 Rheinstetten (DE)
(72) Erfinder: NAUMANN, Dieter, D-1000 Berlin 36 (DE); LABISCHINSKI, Harald, D-1000 Berlin 33 (DE)
(74) Vertreter: KOHLER SCHMID + PARTNER
(86) Internationale Anmeldenummer: DE9000081
(87) Internationale Veröffentlichungsnummer: WO9009453

(56) Entgegenhaltungen:
- EP-A- 0 151 855
- EP-A- 0 164 037
- DE-A- 3 247 891
- Derwent's abstract n 395 55 C/22, SU 690069, publ. week 8022
- Derwent's abstract n 682 45X/36, SU 490820, publ. week 36

## Beschreibung

Die Erfindung betrifft ein Verfahren zur schnellen Detektion von Mikroorganismen in einer Vielzahl von Proben, wie z.B. Urin, Blut, Wasser, Lebensmitteln, pharmazeutischen Rohstoffen und Produkten, wie z.B. Salben, Flüssigpräparaten etc., die in der Regel frei von solchen Mikroorganismen sein sollen oder bei denen bestimmte, festgelegte Grenzwerte an Gesamtkeimzahl oder auch an einzelnen, definierten Vertretern von Mikroorganismen nicht überschritten werden sollen, sowie eine Vorrichtung zur Durchführung des Verfahrens.

Derartige Detektionsarbeiten werden im klinischen Bereich (Sepsis, Harnwegsinfektionen, Liquortestung) und außerklinischen Bereich (Beurteilung der Keimfreiheit bzw. Belastung von Ausgangsmaterialien und Endprodukten, wie z.B. Wasser, Milch, Lebensmittel etc.) in grobem Umfang vorgenommen, da die Ergebnisse derartiger Tests von großer klinischer Bedeutung aber auch von grober Bedeutung für Qualitätskontrolle, Haltbarkeitsbeurteilung und andere Produkt- und Produktionsmitteltests z.B. in industriellen Betrieben sind. Dabei ist wegen der häufigen Möglichkeit des expontiellen Wachstums der zu detektierenden Mikroorganismen und der im klinischen Bereich oft notwendigen sehr frühzeitigen Erkennung von, z.B. Infektionen zur Einleitung therapeutischer Maßnahmen, z.B. im Falle lebensbedrohender Sepsis bzw. im außerklinischen Bereich, z.B. im Falle der Produktion von Pharmaka zur Verhinderung der Unbrauchbarkeit ganzer Chargen, z.B. bei Verwendung verunreinigter Ausgangsmaterialien mit möglicherweise erheblichen ökonomischen Folgen, eine Detektion auch geringer Keimzahlen (z.B. < 10⁵ pro ml bzw. g Probe) in möglichst kurzer Zeit gefordert.

Bei der Durchführung von Detektionsprüfungen bedient man sich in der Regel beim derzeitigen Stand der Wissenschaft entweder Verfahren, die sehr schnell und hochspezifisch sehr kleine Mengen bestimmter, einzelner Gruppen von Mikroorganismen zu detektieren gestatten, und solcher, die weniger schnell und mit vergleichsweise deutlich gröberen Mengen an Mikroorganismen (etwa > 10⁵ Bakterien pro ml Untersuchungsprobe) eine grobe Vielzahl von verschiedenen Mikroorganismen unspezifisch nachweisen können (für eine Übersicht vgl. z.B. in: Bergan, T.: Methods in Microbiology, Vols. 14-16, London, Orlando, San Diego, San Francisco, New York, Toronto, Montreal, Sydney, Tokyo, Sao Paulo: Academic Press (1984); Kipps, T.J., Herzenberg, L.A., in: Habermehl, K.-O.: Rapid Methods and Automation in Microbiology and Immunology, Berlin, New York, Tokyo: Springer Verlag (1985); Toorova, T.P., Antonov, A.S., in: Colwell, R.R. und Grigorova, R.: Methods in Microbiology, Vol. 19, London, Orlando, San Diego, New York, Austin, Boston, Sydney, Tokyo, Toronto: Academic Press (1987); Thronsberry, C., in: Habermehl, K.-O.: Rapid Methods and Automation in Microbiology and Immunology, Berlin, New York, Tokyo: Springer Verlag (1985); Carlberg, D.M., in: Lorian, V.: Antibiotics in Laboratory Medicine, Baltimore, London, Los Angeles, Sydney: Williams & Wilkins (1986)). Zu der ersten Gruppe gehören insbesondere molekularbiologische Nachweismethoden, mit denen die Detektion indirekt durch Nachweis einer Reaktion der Probe, z.B. mit monoklonalen Antikörpern oder aber auch durch DNA/DNA-Hybridisierung u.ä. Techniken erfolgt. Charakteristisch für diese Techniken ist die Notwendigkeit der (kostspieligen) Erzeugung und Bereitstellung von hochspezifischen Reagenzien für jeden einzelnen Typ der zu detektierenden Mikroorganismen. Vorteilhaft bei diesen Verfahren ist jedoch, daß eine Detektion der Mikroorganismen gleichzeitig bereits eine, je nach Spezifität der verwendeten Reagenzien, mehr oder weniger genaue Identifizierung des detektierten Mikroorganismustyps mit beeinhaltet, sofern Kreuzreaktionen mit anderen Organismustypen, die ein häufiges Problem darstellen, vernachlässigt werden. Ihr Einsatz, der ohnehin bisher kaum routinemäßig erfolgt, ist daher absehbar auf einzelne, besonders wichtige (z.B. lebensbedrohende Keime im Falle einer Infektion) Organismengruppen beschränkt. Zu den Vertretern der zweiten Verfahrensgruppe gehören typischer-. weise, z.B. mikrokalorimetrische, Leitfähigkeits/Impedanz- und optische Messungen.

Bei diesen Methoden wird in der Regel die auf Anwesenheit von Mikroorganismen, z.B. Bakterien, zu untersuchende Probe in ein geeignetes, das Wachstum der Organismen förderndes Kulturmedium gegeben und dieses bei möglichst optimalen Wachstumsbedingungen inkubiert. Die Detektion erfolgt dann zum Beispiel durch Nachweis der Wärmeproduktion der Kultur mittels eines Mikrokalorimeters oder der durch das Wachstum der Kultur verursachten Veränderungen der Impedanz der Kulturflüssigkeit mittels geeigneter Leitfähigkeitsmeßsysteme. Im weiteren Sinn gehören zu dieser Verfahrensgruppe auch einfache optische Messungen der Trübung des Kulturmediums oder der lichtoptische Nachweis von Mikroorganismen. Der Vorteil dieser Verfahrensgruppe liegt in der relativ universellen Anwendung auf sehr viele Organismusgruppen und, mit Ausnahme der letztgenannten Verfahrensvariante, in der vergleichsweise einfachen Bedienbarkeit und Automatisierbarkeit. Nachteilig ist dagegen die fehlende Möglichkeit einer parallelen Identifizierung der Mikroorganismen, die, falls erforderlich, separat mit anderen Verfahren durchgeführt werden muß, sowie der u.a. durch Kultivierung bedingte relativ lange Zeitraum zwischen Probenaufgabe und Detektion und die vergleichsweise hohen Nachweisgrenzen (z.B. ca. 10⁵ Zellen/ml Urin im Falle der in der medizinischen Praxis besonders häufigen urologischen Proben).

In der DE-A1-32 47 891 ist ein Verfahren zur Identifizierung von Mikroorganismen auf der Basis der bildanalytischen Auswertung von Infrarotspektren in ausgewählten, kleinen Wellenzahlbereichen beschrieben (vgl. auch Giesbrecht, P., Naumann, D., Labischinski, H., in: Habermehl: Methods and Automation in Microbiology and Immunology, S. 198-206, Berlin, Springer Verlag (1985); Naumann, D., Fijala, V., Labischinski, H., Giesbrecht, P., J. Mol. Struct. 174, 165-170 (1988)). Trotz leistungsfähiger Infrarot-Spektroskope und Infrarot-Mikroskope reicht die Empfindlichkeit dieser Verfahren nicht aus, um Proben auf das Vorhandensein von Mikroorganismen untersuchen zu können.

Der Erfindung liegt die Aufgabe zugrunde, ein Verfahren zur schnellen Detektion einer sehr kleinen Anzahl von Mikroorganismen der eingangs erwähnten Art zu entwickeln, und die Möglichkeit zu schaffen, die unterschiedlichsten Mikroorganismen schnell und sicher nachzuweisen, auch wenn die Anzahl von Mikroorganismen in der zu untersuchenden Probe sehr gering ist.

Diese Aufgabe wird erfindungsgemäß durch die im Patentanspruch 1 angegebenen Maßnahmen gelöst. Durch die Erfindung werden die Vorteile der verschiedenen bisher eingesetzten Verfahrensgruppen, nämlich Nachweis geringer Keimzahlen von Mikroorganismen, Verbindungsmöglichkeit mit Identifizierungsverfahren, geringer Zeitaufwand, breite Anwendbarkeit auf möglichst alle Organismustypen nach einem einheitlichen, leicht zu automatisierenden Verfahren, bei einem Verfahren kombiniert erreicht. Gleichzeitig ist das erfindungsgemäße Verfahren auch in der Praxis der mikrobiologischen Laboratorien im Routinebetrieb effektiv einsetzbar. Das räumlich getrennte Wachstum von Mikrokolonien, ausgehend von einzelnen Individien der Mikroorganismen direkt aus der zu untersuchenden probe auf dem Kulturträger und die Untersuchung einer aus dem Kulturträger gewonnenen Oberflächen-probe nach ortsgetreuer Übertragung, macht das erfindungsgemäße Verfahren gerade zur Untersuchung von proben, die an sich frei von Mikroorganismen sein sollten oder nur eine sehr geringe Keimzahl aufweisen, geeignet und wertvoll.

Die erfindungsgemäß vorgesehene Wachstumszeit von zumindest 6 Generationszeiten der zu detektierenden Organismen beruht auf der Überlegung, daß eine ortsgebundene Konzentrierung des jeweiligen Mikroorganismus auf einem festen Träger für die probe, insbesondere auf einer Agarplatte, eine Anreicherung des Mikroorganismus bis zu einem solchen Grade darstellt, daß die gebildeten Kolonien für die Detektion mit einem Mikroskop, insbesondere IR-Mikroskop, und einem IR-Spektroskop ausreichend groß sind. Durch die geringe Konzentration der Mikroorganismen in der zu untersuchenden Probe, ggf. nach einer etwaigen Verdünnung, wird sichergestellt, daß die einzelnen Mikroorganismen als Individien in einer so großen räumlichen Trennung auf die Oberfläche des festen Trägers aufgebracht werden, daß die daraus entstehenden Kolonien der Mikroorganismen sich gegenseitig nicht beeinträchtigen. Auf diese Weise wird eine Reinzucht der Mikroorganismen im mikroskopischen Bereich unmittelbar aus der Probe durchgeführt, ohne daß eine vorherige oder nachfolgende Isolation der einzelnen Mikroorganismen erforderlich ist. Die Größe der Kolonien der Mikroorganismen soll so sein, daß die jeweilige Kolonie eine Flächenausdehnung besitzt, die einer Anzahl von mindestens ca. 50 Individien, z.B. Bakterien, entspricht. Ausgehend von einer Einzelzelle werden in 6 Generationszeiten 2⁶ gleich 64 Zellen gebildet, deren Flächenausdehnung für die Infrarot-Spektroskopie bereits ausreicht. Bei besonders groben Zellen können auch geringere Zellanzahlen, wie 2⁵ ausreichend sein. Aus Sicherheitsgründen wird jedoch in der Regel mit einer gröberen Flächenausdehnung der Kolonie gearbeitet, die insbesondere durch mindestens 7 - 12 Generationszeiten erreicht wird. 10 Generationszeiten entsprechen etwa 1000 Zellen.

Die Zeitdauer, innerhalb derer das Kolonienwachstum erreicht wird, hängt von der Wachstumsgeschwindigkeit der einzelnen Mikroorganismen ab. Normalerweise ist eine Generationszeit von 20 - 40 Minuten für Proben, die aus dem klinischen Bereich entnommen werden, und eine Generationszeit von ca. einer Stunde, für Wasserproben ausreichend. Um sicher zu gehen, wird die Zeitdauer in der Praxis bei ca. 4 - 10 Stunden gehalten. Die jeweils erforderliche und geeignete Zeitdauer kann auch durch Vorversuche ermittelt werden. Dadurch wird erreicht, daß bei der routinemäßigen Anwendung des Verfahrens mit der kürzestmöglichen Zeitdauer gearbeitet werden kann, um das gewünschte Ergebnis möglichst schnell zu erreichen. Durch die ortsgetreue Übertragung von Oberflächenschichten der erhaltenen Mikrokolonien auf den optischen Träger, wird eine Vermischung von Bestandteilen verschiedener Kolonien vermieden. Dies macht das erfindungsgemäße Verfahren auch zur Enumeration und Identifikation der Mikroorganismen geeignet. Durch die Verwendung von Blenden geeigneten Durchmessers wird eine hohe optische Auflösung und Aussagekraft erreicht.

Eine besonders geeignete Vorrichtung zur Durchführung des erfindungsgemäßen Verfahrens ist Gegenstand der Anspruchs 10. Vorteilhafte Weiterentwicklungen und Merkmale des erfindungsgemäßen Verfahrens und der Vorrichtung ergeben sich aus den Unteransprüchen in Verbindung mit der nachfolgenden Beschreibung. Hierbei können die einzelnen Merkmale jeweils für sich allein oder zu mehreren in Kombination miteinander verwirklicht sein.

Von besonderem Vorteil ist die bevorzugte Verwendung von Infrarot-Mikroskopen, die durch die meßtechnische Weiterentwicklung der Fourier-Transform-Infrarot-Spektroskopie Probenmengen bis unter den Nanogrammm-Bereich hinab reproduzierbar vermessen lassen. Durch die neu entwickelten Techniken der Probenübertragung von Mikrokolonien der Mikroorganismen ohne vorherige Reinkultur, insbesondere direkt auf infrarotdurchlässige oder reflektierende Trägermaterialien, die unmittelbar im optischen und infrarotspektroskopischen Mode des IR-Mikroskops vermessen werden können, wird eine besonders schnelle und sichere Detektion ermöglicht. Überraschenderweise gelingt die Detektion bereits bei der bevorzugten Verwendung eines oder mehrerer auch sehr kleiner Ausschnitte (z.B. nur wenige 100 Wellenzahlen) aus dem Gesamtspektrum, so daß auch Trägermaterialien verwendet werden können, die nur in sehr kleinen Wellenzahlbereichen IR-transparent oder gut reflektierend sind. Als besonders vorteilhaft für die schnelle Detektion erwies sich die Verwendung eines Stempelabdruckverfahrens zur Probenübertragung von einem in der Bakteriologie üblicherweise verwendeten Kulturträger, insbesondere einer Agarkulturplatte, wobei insbesondere ein z.B. kreisrunder Stempel aus IR-transparentem Material geeigneter Dicke, auf zweckmäßige Weise mit Hilfe einer Führung, ggf. auch direkt mit der Hand, an die Kulturplatte kurz (z.B. für weniger als eine Sekunde) angedrückt und wieder abgehoben wird, wobei Teile der auf der Kulturplatte (nach kurzem Bebrüten z.B. einer Urinprobe) sich befindlichen, mit bloßem Auge in der Regel noch nicht wahrnehmbaren Mikrokolonien der ggf. auch verschiedenen Mikroorganismen ortsgetreu auf die Stempelplatte übertragen werden können, so daß die Abdruck-proben auf der Stempelplatte ohne weiteren Präparationsaufwand direkt auf dem Probentisch des FT-IR-Mikroskops vermessen werden können. Diese Vorgehensweise bietet darüber hinaus den Vorteil, daß auf Wunsch des Operators die Bebrütung der originalen Kulturplatten für spätere Kontrollzwecke fortgeführt werden kann. Die Übertragung auf den als optischen Träger dienenden Stempel kann auch durch Andrücken des bewegbaren Kulturträgers gegen die Stempeloberfläche erfolgen.

Als zusätzlicher Vorteil erweist sich die Tatsache, daß das Mikroskop, insbesondere der lichtoptische Modus eines bevorzugt verwendeten IR-Mikroskops (ggf. unterstützt durch Methoden der Bildauswertung bei zum Beispiel aufgesetzter Fernsehkamera mit oder ohne weitere Bildverarbeitung) nicht nur zur Ansteuerung der interessanten Bereiche des Stempels, sondern auch zur Vorbeurteilung der Probe (z.B. nach Morphologie der Mikrokolonien, Farbe, Gestalt der Mikroorganismen (Kokken, Stäbchen etc.), falls gewünscht, mitbenutzt werden kann. Die eigentliche, sichere Detektion erfolgt dann durch Aufnahme der entsprechenden Infrarot-Spektren, die aufgrund der allen Mikroorganismen gemeinsamen grundlegenden chemischen Zusammensetzung (Zellwand, Membran, Protein, Ribosomen, Nukleinsäuren, etc.) eine für alle in Frage kommenden Mikroorganismen prinzipiell ähnliche spektrale Charakteristik aufweisen, so daß die Methodik, wie gewünscht, auf praktisch alle Mikroorganismen ohne Beschränkung angewendet werden kann. Dabei erweist es sich als besonderer Vorteil der Methode, daß die IR-Spektren von Mikroorganismen, ungeachtet ihrer prinzipiellen Ähnlichkeit als Folge der spezifischen chemischen Zusammensetzung der Zellen einzelner Mikroorganismentypen, falls gewünscht, ohne weiteren apparativen Aufwand auch eine Differenzierung und Identifizierung der vermessenen Mikroorganismen durch Vergleich der Spektren mit denen einer geeigneten Referenzdatei bis hin zur Subspezies-Ebene durchführen läßt unter Verwendung einer früher beschriebenen Arbeitsweise (Giesbrecht, P., Naumann, D., Labischinski, H., in: Habermehl: Methods and Automation in Microbiology and Immunology, S. 198-206, Berlin: Springer Verlag (1985); Naumann, D., Fijala, V., Labischinski, H., Giesbrecht, P., J. Mol. Struct. 174, 165-170 (1988)), auf die hier verwiesen wird.

Gemäß der Erfindung ist es, wie bereits erwähnt, möglich, zusätzlich zur Detektion auch eine Identifizierung von Mikroorganismen vorzunehmen. Die erfindungsgemäße Vorrichtung kann jedoch mit Vorteil so ausgestaltet sein, daß alle drei Hauptaufgaben, z.B. Detektion, Identifizierung und ggf. sogar Empfindlichkeitsprüfungen, vorgenommen werden können, so daß ein altes, mit dem bisherigen Stand der Technik aber nicht realisierbares Ziel einer einheitlichen Technik mit prinzipiell einheitlichem Arbeitsgang für beliebige Mikroorganismen nunmehr verwirklicht werden kann. In diesem Zusammenhang erweist es sich als sehr vorteilhaft, den gesamten Verfahrensablauf von der Probenaufbereitung über die Probenvermessung bis zur Ausgabe des Ergebnisses vollständig zu automatisieren, falls gewünscht sogar unter Einbeziehung einer nachfolgenden Weiterverarbeitung des Ergebnisses, etwa im Sinne einer Identifizierung oder Empfindlichkeitsprüfung, da das Verfahren für alle in Frage kommenden Mikroorganismen im Prinzip einheitlich durchgeführt werden kann und die Technik der Spektrenregistrierung durch die FT-IR-mikroskopische Technik von vornherein ein digitales und damit leicht auf elektronischem Wege zu verarbeitendes Meßergebnis liefert.

Bei der Durchführung einer bevorzugten Ausführungsform des erfindungsgemäßen Verfahrens wird die zu untersuchende Probe, wie z.B. Urin, Blut, Liquor, Wasser, wäßrige Auszüge aus Lebensmitteln, Salben, Arzneimitteln etc., in der in der Mikrobiologie üblichen Weise auf eine feste Nährbodenplatte in der aus der Mikrobiologie bekannten Art so verdünnt plattiert, daß die ggf. in der Probe vorhandenen Mikroorganismen zu Kolonien vereinzelt wachsen. Nach einer kurzen Kultivierungszeit von etwa 4-10 Stunden (in Abhängigkeit von der Probenart und der Generationszeit der zu detektierenden Mikroorganismen), in der sich auf der Platte mit dem Auge noch nicht detektierbare Mikrokolonien aus zum Beispiel ca. 10³ Organismen gebildet haben, erfolgt der Transfer von der Kulturplatte auf den im gewünschten Wellenzahlbereich durchlässigen oder reflektierenden Träger, z.B. durch Auflegen einer Probenträgerscheibe von z.B. ca. 2 mm Dicke und 3 cm Durchmesser aus z.B. ZnSe, poliertem Stahl etc. und anschließendes Wiederanheben, so daß sich auf dem Probenträger ein ortsentsprechender Abdruck von anhaftenden Mikroorganismen von der Kulturplatte ergibt. Der Probenträger wird anschließend manuell oder auch automatisch gesteuert auf den Probentisch des Mikroskops, insbesondere IR-Mikroskops, überführt. An dieser Stelle des Verfahrens sind je nach Wunsch des Experimentators und gegebener Detailausstattung des Mikroskops mehrere Varianten möglich. Im einfachsten Falle kann die Probenplatte im optischen Mode des Mikroskops manuell/visuell inspiziert werden. Aus der Zahl der optisch erkennbaren Mikrokolonien kann (unter Berücksichtigung eventueller Verdünnungsfaktoren je nach Probenverdünnung in der in der Mikrobiologie üblichen Weise) bereits eine erste Abschätzung über die in der Probe vorhandene Keimzahl erfolgen, aus der Kolonie-Morphologie (Größe, Farbe, Oberflächenbeschaffenheit etc.) eventuell auch bereits eine grobe Abschätzung über eventuell unterschiedliche Organismusgruppen. Sind zu diesem Zeitpunkt keine Mikrokolonien erkennbar, kann das Verfahren an dieser Stelle bereits mit dem Ergebnis "negativ" beendet werden. Die eigentliche Detektion erfolgt dann im infrarotspektroskopischen Mode des Mikroskops, in dem von allen, bzw. von einer repräsentativen Zahl der auf dem Probenträger befindlichen Mikrokolonien nacheinander unter Zuhilfenahme der in jedem IR-Mikroskop integrierten Blendensysteme die Infrarot-Spektren der entsprechenden Mikrokolonien registriert werden. Nur solche optisch vorher erfaßten Mikrokolonie-Abdrücke, deren Spektren mit der für Mikroorganismen typischen chemischen Zusammensetzung (vor allem Proteine, Lipide, Nukleinsäuren, Polysaccharide) kompatibel sind, werden als endgültig detektiert akzeptiert. Auf diese Weise wird verhindert, daß im optischen Mode erkannte Abdruck-Stellen, die z.B. von anorganischen Verschmutzungen (Staub etc.) oder auch z.B. von Übertragungen von Medien- oder Agarkomponenten stammen, fehlerhaft zum Ergebnis beitragen. Die erhaltenen Spektren können, falls gewünscht, im Sinn einer Verfahrensvariante, sofort anschließend oder auch zu einem späteren Zeitpunkt durch Abgleich mit einer Spektrenreferenzdatei in dem oder den gewünschten, auch sehr kleinen Spektralbereich(en), nach den veröffentlichten Verfahrensgrundsätzen (s.o.) auch hinsichtlich einer Identifizierung der Mikroorganismen herangezogen werden. Um, falls gewünscht, eine größere Unabhängigkeit des Verfahrens von menschlichen Eingriffen in den Verfahrensgang, wie oben prinzipiell beschrieben, zu erreichen, sind eine Reihe weiterer Verfahrensmodifikationen möglich. So können z.B. die vorgesehenen Transferschritte (also a) Auftragung der probe auf die Kulturplatte, b) Übertragung der beimpften Kulturplatte in einen Inkubator, c) Stempelabdruck zur Übertragung der Mikrokolonien vom Kulturträger auf den optischen Probenträger, d) ggf. Überführung des Probenträgers auf den IR-Mikroskop-Probentisch und e) Abtransport der probe nach erfolgter Messung) leicht mechanisiert und/oder automatisiert unter Zuhilfenahme üblicher mechanischer und/oder elektronischer Komponenten vorgenommen werden. Eine darüber hinausgehende, vollständige automatische Verfahrensabwicklung kann erreicht werden durch Hinzufügen eines Bildverarbeitungssystems, bestehend aus einer auf dem IR-Mikroskop angeflanschten Kamera, einem elektronisch gesteuerten X-Y-Kreuztisch als Mikroskoptisch, einer elektronisch gesteuerten Umschalteinheit zwischen optischen und IR-mikroskopischem Mode des Mikroskops, einer Steuer- bzw. Rechnereinheit (z.B. Personalcomputer mit entsprechenden Interfaces oder auch andere Rechnersysteme) und einem oder mehreren entsprechenden Auswerteprogrammen. Zweckmäßigerweise kann die Rechner- und Steuereinheit einschließlich Auswerteprogrammen in die der Steuerung des FT-IR-Geräts einschließlich IR-Mikroskops dienenden, von vornherein vorhandenen Rechner-/Steuereinheit integriert werden.

Ein vollautomatischer Verfahrensablauf gestaltet sich dann in folgender Weise: Nach dem Transfer des die Mikrokolonien-Abdrücke enthaltenden Probenträgers auf den X-Y-Tisch des IR-Mikroskops kann eine Abbildung des Trägers im optischen Mode des Mikroskops von der Kamera aufgenommen und digitalisiert (in zumindest 256 x 256 Pixel, z.B. 64 Graustufen) an die Rechner-/Steuereinheit weitergegeben werden. Dort können mittels eines Teilprogramms die X-Y-Positionen möglicher Mikrokolonien-Abdrücke durch Auswertung der digitalen Graustufenverteilung des Bildes, z.B. mit einem Peaksuch-Algorithmus, ermittelt werden. Darauffolgend kann von der Steuereinheit eine geeignete Blende (Durchmesser z.B. 30-80 µm) eingefahren, eine Positionierung auf die Ortskoordinaten des ersten vermuteten Mikrokolonien-Abdrucks vorgenommen und das Infrarot-Spektrum an dieser Stelle registriert werden. Dieser Vorgang kann wiederholt werden, bis alle vorher ermittelten X-Y-Positionen abgearbeitet sind. Die Steuereinheit kann anschließend den Aufruf eines weiteren Teilprogramms veranlassen, das sämtliche so registrierten Spektren auf ihre Kompatabilität mit Mikroorganismen-Spektren hin überprüft. Dies kann z.B. über eine einfache Kreuzkorrelation ggf. mit vorhergehender Filterung der Spektren mit Referenzspektren von Mikroorganismen erreicht werden, da für jeden einzelnen aber auch kombinierten Spektralbereich bei vorgegebener Filtermethode ein Threshold-Wert des Korrelationskoeffizienten zwischen Mikroorganismus-Spektren existiert, dessen Unterschreitung die vermutete Mikrokolonie als anorganische oder organische Verunreinigung des Probenträgers identifiziert. Dabei reicht eine Referenzdatei von 5-20 typischen Mikroorganismen-Spektren (z.B. Staphylokokken, Streptokokken, Clostridien, Pseudomonaden, Enterobacteriaceen, Salmonellen, Legionellen, Bazillen) zur sicheren Diskriminierung zwischen Mikroorganismen und Verunreinigung aus. Selbstverständlich können für spezielle Anwendungen auch Peaktabellen oder z.B. eine Auswertung mittels bekannter Methoden der multivariaten Statistik herangezogen werden. Die Zahl der in der Probe vorhandenen Mikroorganismen kann anschließend von der Steuer-/Recheneinheit aus der Anzahl der als Mikrokolonien-Abdrücke bestätigten Positionen, ggf. unter Berücksichtigung entsprechender Verdünnungsfaktoren z.B. als Keimzahl/ml Probe berechnet und ausgegeben werden. Mit Hilfe der Steuer- und Recheneinheit kann natürlich, falls gewünscht, bei entsprechender Programmierung auch eine nachfolgende Identifizierung der detektierten Mikroorganismen durch Vergleich mit einer Referenzdatei vorgenommen werden, die aus unter gleichen Bedingungen gemessenen Referenzspektren besteht. Die benötigten Programmteile bauen auf dem Fachmann wohlbekannten Algorithmen auf und können selbst erstellt oder aus einer Vielzahl von allgemein erhältlichen Programmsystemen (erfolgreich verwendet in diesem Zusammenhang wurde z.B. das Programm IMAGIC, siehe M. van Heel, W. Keegstra, Ultramicroscopy 7, 113, 1981) übernommen werden.

Insgesamt erlaubt das neue Verfahren zur Detektion von Mikroorganismen in der geschilderten Weise einen raschen qualitativen und quantitativen Nachweis von Mikroorganismen prinzipiell aller Art mit dem weiteren Vorteil der zusätzlichen Identifizierung mit ein und derselben Grundapparatur in einer Zeit von ca. 4-10 Stunden oder weniger nach Probenabgabe ohne vorherige Reinkultur, wobei diese Zeitdauer fast vollständig durch die notwendige Inkubationszeit bis zum Erhalt von Mikrokolonien bestimmt wird, da die weiteren Verfahrensschritte lediglich Minuten bis weniger als eine Stunde bei geeigneter Verfahrensdurchfuhrung in Anspruch nehmen.

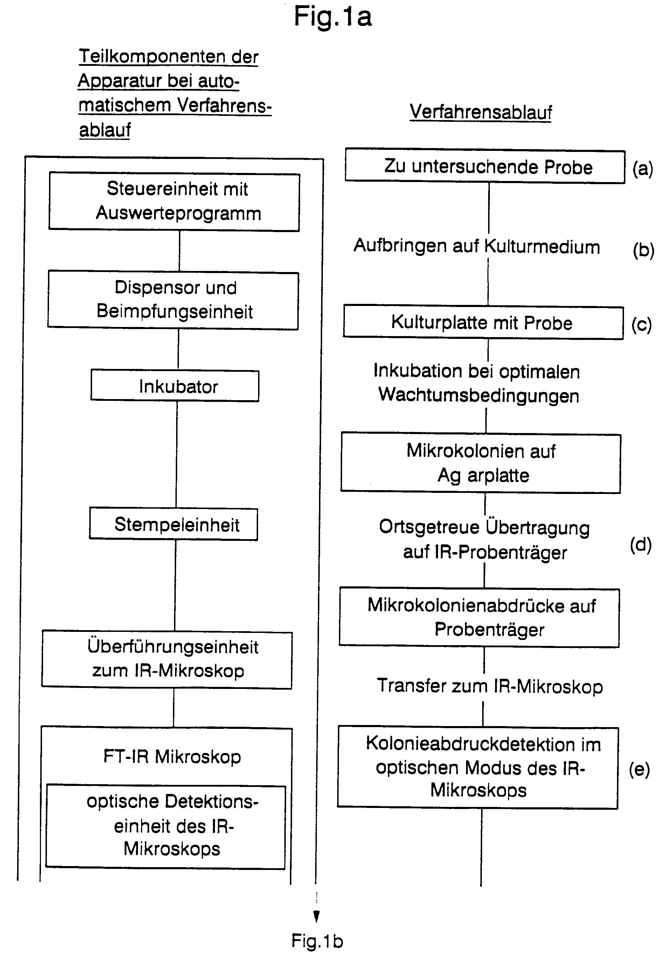

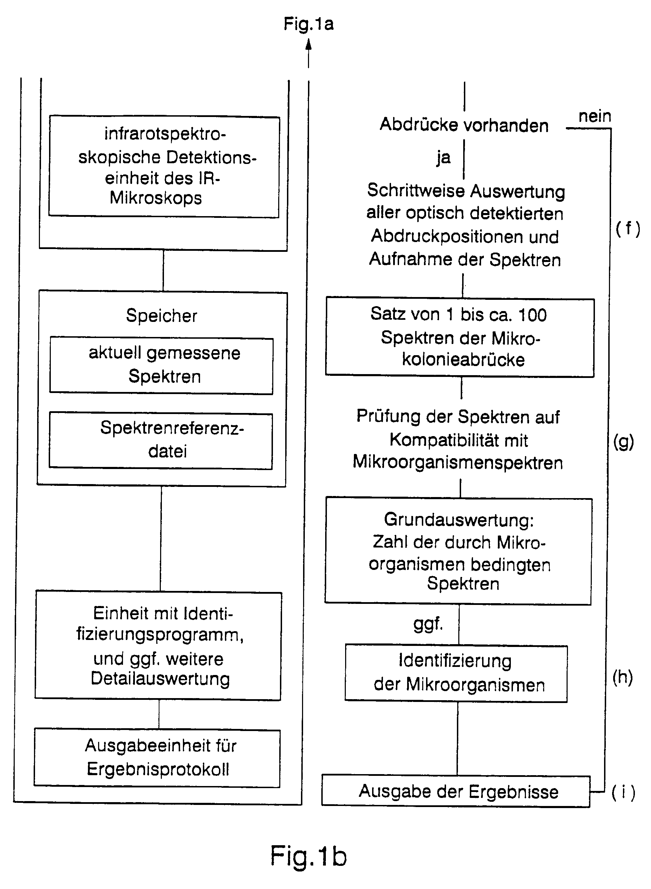

Eine Grobübersicht über den gesamten Verfahrensablauf von bevorzugten Ausführungsformen zeigt Fig. 1 in schematischer Darstellung.

### Beispiel 1

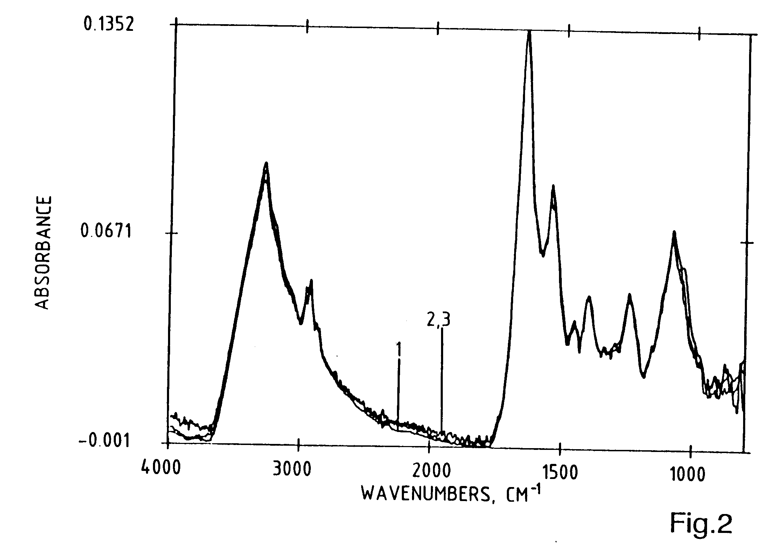

Dieses Beispiel 1 soll im Zusammenhang mit Fig. 2 die außerordentliche Sensitivität des neuen Verfahrens belegen, ohne den gesamten Verfahrensablauf zu beschreiben. Fig. 2 zeigt drei mit einem Infrarot-Mikroskop A 560, Fa. Bruker, Karlsruhe, gekoppelt an ein IFS 48 FT-IR-Spektrometer derselben Firma auf folgende Weise erhaltene Spektren:

100 ul einer 10³ Keime/ml enthaltende Kultur von Klebsiella pneuminiae, ATCC 13882 in Peptonwasser wurde auf eine Pepton-Agarplatte ausplattiert und für 8 Stunden bei 37 °C inkubiert (ca. 24 Generationszeiten). Der Abdruck der Bakterien erfolgte durch vorsichtiges Auflegen und Wiederabheben eines polierten BaF₂ Probeträgers von 40 mm Durchmesser und 3 mm Dicke. Der dabei übertragene, in der Regel (insbesondere bei kürzeren Inkubationszeiten) aus 1-3 Bakterienschichten bestehende Bakterienfilm trocknet innerhalb weniger Sekunden bei Raumbedingungen ohne Anwendung von Vakuum oder Trocknungsmitteln an. Die Inspektion des Probenträgers im optischen Mode des IR-Mikroskops (Objektiv 15x, Cassegranian, Okular 20x) ergab einen ausgedehnten, dünnen, 1-3lagigen Bakterienfilm über praktisch die gesamte Probenträgerfläche. Durch die Wahl geeigneter Kreisblenden wurde erreicht, daß jeweils ca. 10⁴ (Spektrenzug 1 in Fig. 2, Blende 80 µm Durchmesser), ca. 10³ (Spektrenzug 2, Blende 40 µm Durchmesser) bzw. ca. 10² (Spektrenzug 3, Blende 20 µm Durchmesser) Mikroorganismen zum Spektrum beitragen. Sämtliche Spektren wurden in Transmission vermessen, 512 Scans wurden bei einer spektralen Auflösung von 8 cm⁻¹ akkumuliert. Inspektion der Fig. 2 zeigt eindeutig, daß bereits bei ca. 10² Mikroorganismen (entspricht weniger als 0,1 ng Substanz) klar als Mikroorganismen-Spektren zu detektierende Spektrensignale, hier im Bereich von ca. 4000 - 900 Wellenzahlen dargestellt, zu erhalten sind. Aufgrund des relativ hohen Rauschanteils (vgl. Spektrenzug 3 in Fig. 2) ist jedoch das Arbeiten mit zumindest ca. 10³ Mikroorganismen dann vorzuziehen, wenn eine möglichst tiefgehende, z.B. Spezies-Identifizierung sich an die Detektion mit dem gleichen Datensatz anschließen soll. Aus diesem typischen Meßbeispiel ist abzulesen, daß die Inkubationszeit der Probe auf der Kulturplatte zumindest ca. 6 bis 12 Generationszeiten betragen sollte, um aus jedem auf der Kulturplatte durch entsprechend verdünntes Auftragen vereinzelten Mikroorganismus eine Mikrokolonie von ca. 200 - 4000 Organismen entstehen zu lassen. Somit ergibt sich z.B. für Enterobacteriaceen (typische Generationszeit auf üblichen Medien ca. 20 Minuten) eine untere Grenze für den Inkubationszeitraum von weniger als 4 Stunden, für Staphylokokken (Generationszeit auf üblichen Medien z.B. 30 Minuten) von ca. 5 Stunden etc..

### Beispiel 2

Dieses Beispiel zeigt einen typischen Verfahrensablauf bei manueller Versuchsdurchführung am Beispiel einer wäßrigen Probe, die künstlich mit 10³ Zellen/ml von Staphylococcus aureus Stamm Pelzer versetzt wurde. 100 µl dieser Probe wurden auf eine Pepton-Agarplatte (100 mm Durchmesser) aufgetragen und mit Hilfe eines Drigalski-Spatels verteilt. Nach 8-stündiger Inkubationszeit bei 37 °C in einem Brutschrank wurden die (mit bloßem Auge noch nicht erkennbaren) Mikrokolonien in der im Beispiel 1 beschriebenen Weise (Stempelabdruck) auf eine 40 mm BaF₂ Platte ortsgetreu übertragen.

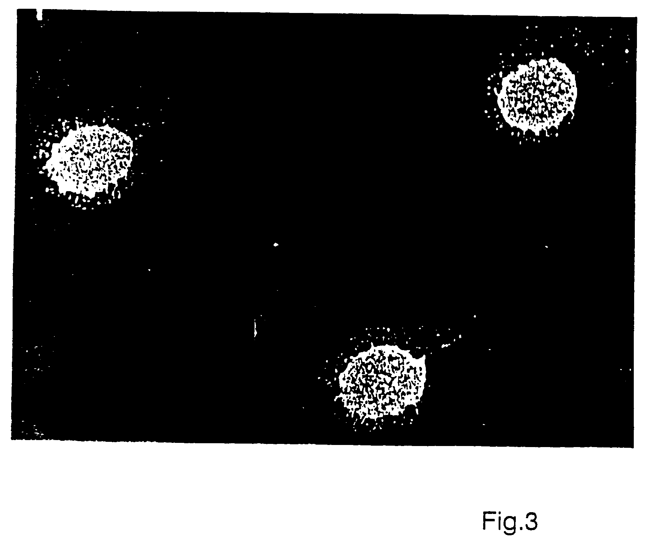

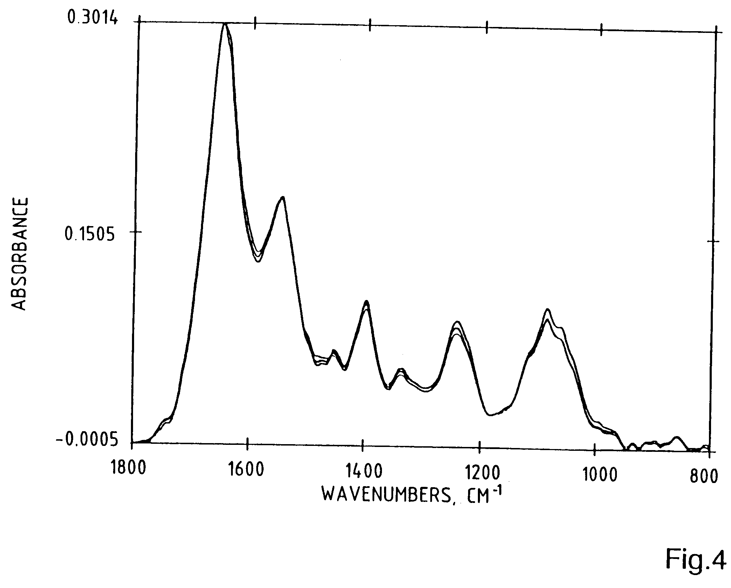

Fig. 3 zeigt eine vergrößerte Aufnahme (250x) eines Ausschnitts des Probenträgers. Die von den in Fig. 3 gezeigten drei Mikrokolonie-Abdrücken erhaltenen Spektren im Bereich zwischen 1800 und 800 cm⁻¹ sind in Fig. 4 dargestellt. Schon der visuelle Vergleich der drei Spektren zeigt die ausgezeichnete Reproduzierbarkeit der Methode bei Erfassung unterschiedlicher Mikrokolonien des gleichen Mikroorqanismus. Zur quantitativen Bestätigung wurde durch Kreuskorrelation der sogenannte Differenzzierungsindex D (D= (1- α) · 1000,α = Pearson-scher Korrelationskoeffizient) bestimmt. Er ergab sich bei den hier gezeigten ebenso wie bei weiteren Spezies analog durchgeführten Messungen zu D ≈ 8 unter Einbeziehung von Messungen von unabhängig präparierten Proben, die zu unterschiedlichen Zeiten auf Agarplatten unterschiedlicher Chargen desselben Nährmediums angefertigt wurden. Somit bestimmt der D-Wert von ca. 8 (entsprechend einem Korrelationskoeffizient von 0.992!) das überraschend gute Reproduktionsniveau des Verfahrens und liefert für den Fall einer gewünschten anschließenden Differenzierung bzw. Identifizierung der detektierten Mikroorganismen den Threshold-Wert zur Unterscheidung verschiedener Mikroorganismen. Im beschriebenen Beispiel wurden auf der Trägerplatte 17 Mikrokolonien-Abdrücke über ihr Infrarot-Spektrum detektiert. Aus diesem Wert errechnet sich unter Berücksichtigung der eingesetzten Probenmenge (100 µl) und des Flächenverhältnisses von Agarplatte zu Probenträgerfläche (6.26 : 1) eine detektierte Keimzahl von 1.06x10³ in exzellenter Übereinstimmung mit dem eingesetzten Wert.

### Beispiel 3

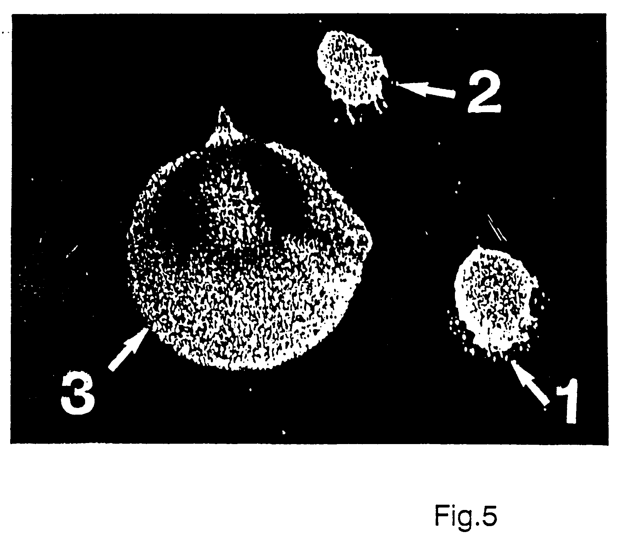

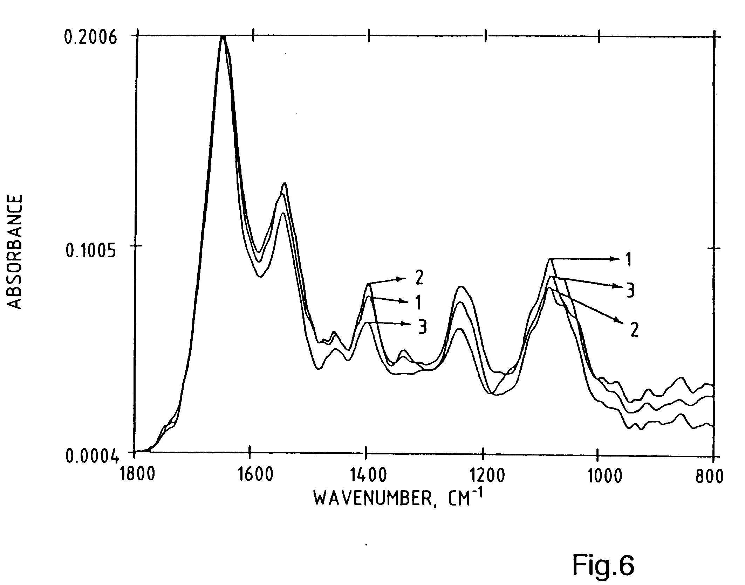

Dieses Beispiel illustriert die Vorgehensweise einer sich an die Detektion anschließenden Identifizierung der gefundenen Mikroorganismen:
100 µl einer Wasserprobe mit je 10³ Keimen pro ml von Staphylococcus aureus, Staphylococcus xylosus und Klebsiella pneumoniae wurden wie in Beispiel 2 auf eine Pepton-Agarplatte aufgebracht, nach einer Inkubationszeit von 8 Stunden und mit der identischen Stempeltechnik auf den gleichen Probenträger, wie in Beispiel 2 beschrieben, aufgebracht. Fig. 3 zeigt einen 250x vergrößerten Ausschnitt des Probenträgers. Wegen der orts- und damit auch flächengenauen Übertragung der Mikrokolonien ergibt bereits die Betrachtung im optischen Mode des Mikroskops auch für einen bakteriologisch nur wenig geschulten Experimentator Hinweise auf die Beteiligung mehrerer Organismen, denn die unterschiedlichen Mikrokoloniengrößen, wie aus Fig. 4 ersichtlich, weisen bereits auf drei verschiedene Mikroorganismen hin. Bei höherer Vergrößerung kann auch die Einzelbakterienmorphologie (z.B. Kokken oder Stäbchen) mit herangezogen werden. Dies stellt jedoch für den Experimentator nur ein mögliches zusätzliches Hilfsmittel dar, denn im Verfahrensablauf erfolgt die Detektion durch Registrierung der entsprechenden Infrarot-Spektren. Die für die in Fig. 5 mit 1, 2 und 3 bezeichneten Mikrokolonien-Abdrücke erhaltenen Spektren im Infrarotmodus der FT-IR-Mikroskops zeigt Fig. 6. Alle Spektren zeigen über den charakteristischen Probenverlauf die Präsenz von Mikroorganismen an. Der Vergleich der in Fig. 6 dargestellten Spektrenzüge mit denen einer Referenzdatei aus 100 Spektren von Bakterien aus 40 verschiedenen Spezies ergab auf der Basis der niedrigsten D-Werte, berechnet aus den 1. Ableitungen der Spektren im Wellenzahlbereich von 1500-900 cm⁻¹, eine für alle Mikrokolonie-Abdrücke speziesgenaue Identifizierung.

### Beispiele und Bemerkungen zu Fig. 1

(a) z.B. Urin, Liquor, Blut, Lebensmittel, Wasser, etc., ggf. nach entsprechender Aufbereitung und Verdünnung
(b) z.B. Agarplatte mit Blutzusatz, Pepton o.ä., ggf. Selektiv- oder Elektivmedium
(c) z.B. 20-40 Grad Celsius, pH 4-9, für z.B. 4-10 Std. bzw. etwa 8-20 Generationszeiten der Mikroorganismen
(d) z.B. Stempelabdruck auf ZnSe, BaF₂-Scheiben, Polymerfilm, Aluplättchen, polierte Stahlplättchen etc.
(e) z.B. manuelle, visuelle oder automatische Abrasterung des Probenträgers, Registrierung und ggf. elektronische Speicherung von Abdruckpositionskoordinaten, ggf. unter Heranziehen von Bildverarbeitungstechniken, die Auskunft über Zahl, Position und Größe der Kolonieabdrücke liefern
(f) z.B. seriell, manuell oder automatisch gesteuertes Anfahren der im vorigen Schritt ermittelten Abdruckkoordinaten, Einschwenken einer geeigneten Blende im Bereich von ca. 10 - 200 um und Aufnahme der Spektren im Wellenzahlbereich von z.B. 5000 - 500 im mittleren Infrarot
(g) z.B. optische Spektrenbeurteilung durch Experimentator, Berechnung von Korrelationskoeffizienten zu Referenzspektren, multivariate Analyse der Spektren etc.
(h) ggf. Identifizierung durch Vergleich mit Referenzdaten
(i) ggf. unter Berücksichtigung von Probenverdünnung etc., Zusatzergebnisse wie Identifizierung, Kolonienform etc.

## Patentansprüche

1. Verfahren zur schnellen Detektion von Mikroorganismen, wie Bakterien und Pilzen, in Proben, dadurch gekennzeichnet, daß eine auf Mikroorganismen zu untersuchende Probe
a) auf einen in der Mikrobiologie verwendeten im wesentlichen festen Kulturträger in einer Verdünnung aufgetragen wird, die das Wachstum vorhandener Mikroorganismen in örtlich getrennten Kolonien ermöglicht,
b) ein kurzzeitiges Wachstum von zumindest 6 Generationszeiten der zu detektierenden Organismen zu örtlich getrennten Mikrokolonien unter den in der Mikrobiologie üblichen Bedingungen veranlaßt wird,
c) Mikrokolonien aufweisende Oberflächenbereiche des Kulturträgers auf einen im gewünschten Spektralbereich entweder zumindest teildurchlässigen oder reflektierenden optischen Träger ortsgetreu übertragen werden,
d) die ortsgetreue Übertragung der Mikroorganismen vom Kulturträger in direkter Weise auf einen Stempel vorgenommen wird, dessen Stempelfläche aus einem, im gewünschten Wellenbereich durchlässigen oder reflektierenden Material besteht,
e) einzelne Kolonien der übertragenen Mirkoorganismen in einem Mikroscop lokalisiert werden und
f) von den übertragenen Mikroorganismen-Kolonien einzeln mittels eines Infrarotmikroskops im Transmission- oder Reflektionsmodus unter Verwendung von Blenden im Durchmesserbereich von etwa 10 - 200 µm sequentell IR-Spektren aufgenommen und ausgewertet werden.

2. Vefahren nach Anspruch 1, dadurch gekennzeichnet, daß ein Ausschluß von auf den Träger übertragenen Bestandteilen, die nicht Mikroorganismen repräsetieren, durch einen Vergleich der erhaltenen Spektren mit Spektren von Referenzmikroorganismen durchgeführt wird.

3. Verfahren nach einem der vorhergehenden Ansprüche, dadurch gekennzeichnet, daß die Lokalisierung der Mikroorganismen-Kolonien in einer optischen oder IR-spektroskopischen Betriebsart des Infrarotmikroskops durchgeführt wird.

4. Verfahren nach einem der vorhergehenden Ansprüche, dadurch gekennzeichnet, daß die Mikroorganismenprobe durch einfachen Abdruck vom Kulturträger auf einen Stempel übertragen wird und der Stempel dann als optischer Probenträger in das IR-Mikroskop zur Vermessung der Spektren überführt wird.

5. Verfahren nach einem der vorhergehenden Ansprüche, dadurch gekennzeichnet, daß die Infrarotspektren in einem oder auch mehreren, ggf. sehr kleinen Wellenzahlintervallen von weniger als 300 cm⁻¹, oder auch nur einzelne, selektive, für bestimmte Mikroorganismen charakteristische Absorptionsbanden oder Bandenkonturen im NIR-, MIR- oder FIR-Bereich des Infrarotspektrums gemessen werden.

6. Verfahren nach einem der vorhergehenden Ansprüche, dadurch gekennzeichnet, daß die Auswertung der Spektren zur Bakteriendetektion mittels mathematischer Techniken der Datenreduktion,durch Methoden der multivariaten Statistik- oder Berechnung von Korrelationskoeffizienten zu Referenzspektren durchgeführt wird.

7. Verfahren nach einem der vorhergehenden Ansprüche, dadurch gekennzeichnet, daß neben der reinen Detektion von Mikroorganismen zusätzlich eine Identifikation derselben durch Vergleich mit Spektrenreferenzdateien vorgenommen wird.

8. Verfahren nach einem der vorhergehenden Ansprüche, dadurch gekennzeichnet, daß zur Enumeration der Mikroorganismen die Anzahl der pro Flächeneinheit gefundenen Mikroorganismen-Kolonien bestimmt und auf die Probe zurückgerechnet wird.

9. Verfahren nach Anspruch 8, dadurch gekennzeichnet, daß die Zählung verschiedenartiger Kolonien getrennt erfolgt.

10. Vorrichtung zur Durchführung des Verfahrens nach einem der vorhergehenden Ansprüche, gekennzeichnet durch Kopplung oder Integration
a) einer Beimpfungseinheit zur Aufgabe der Proben auf Kulturträger,
b) einer Inkubatoreinheit zum Bebrüten der Kulturen,
c) einer Übertragungseinheit zum ortsgetreuen Übertragen von gebildeten Mikrokolonien vom Kulturträger auf einen optischen Träger in Form eines Stempels, dessen Stempelfläche aus einem im gewünschten Wellenbereich durchlässigen oder reflektierenden Material besteht,
d) einer Einheit zum Lokalisieren von Mikroorganismen-Kolonien mittels eines Mikroskops,
e) eines IR-Mikroskops und
f) einer Einheit zur kontrollierten Steuerung der einzelnen Funktion der Apparatur.

11. Vorrichtung nach Anspruch 10, gekennzeichnet weiterhin durch eine Überführungseinheit zur Übertragung des optischen Trägers auf den Objekttisch eines Mikroskops, eine mechanische Einheit zum Abrastern der übertragenen Probe unter Nutzung des optischen Modus eines IR-Mikroskops oder einer Detektionseinheit zur Erfassung von Mikrokolonien-Abdrücken auf den optischen Träger im optischen und infrarotspektroskopischen Modus eines IR-Mikroskops.

12. Vorrichtung nach einem der Ansprüche 10 oder 11, dadurch gekennzeichnet, daß die Steuereinheit der Apparatur parallel oder alternativ zur Detektion auch zur Steuerung einer Identifizierung eingerichtet ist.

## Claims

1. Method for the rapid detection of microorganisms, such as, for example, bacteria and fungi, in samples, characterized in that a sample to be tested for the presence of microorganisms
a) is applied to an essentially solid culture carrier, used in microbiology, in a dilution which enables the growth of microorganisms present into locally separated colonies,
b) a short-term growth of at least 6 generation times of the organisms to be detected, to form locally separated microcolonies, is initiated under the conditions customary in microbiology,
c) regions of the surface of the culture carrier on which microcolonies are present are transfer locality-true to an optical carrier which is either at least partially transparent or reflective in the desired spectral range,
d) the locality-true transfer of the microorganisms from the culture carrier takes place directly to a stamp, whose surface is made from a material which is transparent or reflecting in the desired wave band,
e) individual colonies of the transfer microorganisms are located under a microscope and
f) IR spectra of the transferred microorganism colonies are sequentially recorded individually by means of an infra-red. microscope in the transmission or reflection mode, using apertures in the diameter range of about 10-200 µm, and evaluated.

2. Method as claimed in claim 1, characterized in that any constituents transferred to the carrier which do not represent microorganisms are excluded by comparison of the spectra obtained with spectra of reference microorganisms.

3. Method as claimed in any of the preceding claims, characterized in that the localization of the microorganism colonies is carried out using the infra-red microscope in an optical or IR-spectroscopic mode.

4. Method as claimed in one of the preceding claims, characterized in that the microorganism sample is transferred from the culture carrier to a stamp by simple impression and the stamp is then transferred as optical sample carrier into the IR microscope for measurement of the spectra.

5. Method as claimed in any of the preceding claims, characterized in that the infra-red spectra are measured in one or more wavenumber ranges, which may be very small, being less than 300 cm⁻¹, or only individual, selective absorption bands or band profiles in the NIR, MIR or FIR region of the infra-red spectrum which are characteristic for specific microoganismens.

6. Method as claimed in any of the preceding claims, chacterized in that the evaluation of the spectra for the detection of bacteria is carried out by means of mathematical techniques for data reduction, by multi-variance statistics methods or calculated of correlation coefficients relative to reference spectra.

7. Method as claimed in any of the preceding claims, characterized in that in addition to the pure detection of microorganisms, an identification thereof is additionally carried out by comparison with reference spectra databases.

8. Method as claimed in any of the preceding claims, characterized in that for enumeration of the microorganisms the number of microorganism colonies found per surface unit is determined and back-calculated to the sample.

9. Method as claimed in claim 8, characterized in that the different types of colonies are counted separately.

10. Device for carrying out the method as claimed in any of the preceding claims, characterized by coupling or integration of
a) an inoculation unit for applying the samples to culture carriers,
b) an incubator unit for incubating the cultures,
c) a transfer unit for the locality-true transfer of microcolonies formed from the culture carrier to an optical carrier in the form of a stamp, whose surface is made from a material transparent or reflecting in the desired wave band,
d) a unit for locating microorganism colonies by means of a microscope,
e) an IR microscope and
f) a unit for regulated control of the individual functions of the apparatus.

11. Device as claimed in claim 10, characterized by a transfer unit for transferring the optical carrier to the stage of a microscope, a mechanical unit for scanning the transferred sample using the optical mode of an IR microscope or a detection unit for detecting microcolony impressions on the optical carrier in the optical and infra-red spectroscopic mode of an IR microscope.

12. Device as claimed in one of the claims 10 or 11, characterized in that the control unit of the apparatus is also equipped for controlling an identification process, in parallel with or as an alternative to the detection.

## Revendications

1. Procédé de détection rapide de microorganismes, tels que des bactéries et des champignons, dans des échantillons, caractérisé en ce qu'un échantillon à examiner quant aux micro-organismes
a) est appliqué sur un support de culture sensiblement solide, utilisé en microbiologie, dans une dilution qui permet la croissance des micro-organismes présents en colonies localement séparées,
b) une croissance de courte durée d'au moins 6 temps de génération des organismes à détecter est provoquée dans les conditions courantes en microbiologie, en vue de former des microcolonies localement séparées,
c) des zones superficielles, présentant des microcolonies, du support de culture sont transférées d'une manière fidèle quant à la localisation sur un support optique au moins partiellement transparent ou réflecteur dans la gamme spectrale souhaitée,
d) le transfert, fidèle quant à la localisation, des micro-organismes est effectué depuis le support de culture d'une manière directe sur un poinçon dont la surface de poinçon est constituée d'une matière transparente ou réflectrice dans la gamme d'ondes souhaitée,
e) des colonies individuelles des micro-organismes transférés sont localisées dans un microscope, et
f) des spectres IR des colonies de micro-organismes transférés sont absorbés individuellement de manière séquentielle au moyen d'un microscope à infrarouges dans le mode transmission ou réflexion, en utilisant des ouvertures dans la gamme de diamètres d'environ 10 à 200 µm, et sont évalués.

2. Procédé suivant la revendication 1, caractérisé en ce qu'une exclusion des éléments, qui sont transmis sur le support et qui ne représentent pas des micro-organismes, est effectuée par une comparaison entre les spectres obtenus et les spectres de microorganismes de référence.

3. Procédé suivant l'une des revendications précédentes, caractérisé en ce que la localisation des colonies de micro-organismes est effectuée dans un mode de fonctionnement optique ou IR-spectroscopique du microscope aux infrarouges.

4. Procédé suivant l'une des revendications précédentes, caractérisé en ce que l'échantillon de micro-organismes est transféré du support de culture sur un poinçon par une simple application et en ce que le poinçon est ensuite passé, comme support d'échantillon optique, dans le microscope aux IR pour la mesure des spectres.

5. Procédé suivant l'une des revendications précédentes, caractérisé en ce que les spectres infrarouges sont mesurés dans un ou même plusieurs intervalles de nombres d'ondes, éventuellement très petits, inférieurs à 300 cm⁻¹, ou encore en ce que seulement des bandes d'absorption ou contours de bandes individuelles, sélectives, caractéristiques pour des micro-organismes déterminés sont mesurées dans la zone NIR, MIR ou FIR du spectre infrarouge.

6. Procédé suivant l'une des revendications précédentes, caractérisé en ce que l'évaluation des spectres pour la détection des bactéries est effectuée au moyen de techniques mathématiques de la réduction des données, par des méthodes de la statistique à multivariance ou par calcul de coefficients de corrélation par rapport à des spectres de référence.

7. Procédé suivant l'une des revendications précédentes, caractérisé en ce que, en plus de la détection pure des micro-organismes, on effectue en supplément une identification de ceux-ci par une comparaison avec des données de référence spectrales.

8. Procédé suivant l'une des revendications précédentes, caractérisé en ce que, pour l'énumération des micro-organismes, on détermine le nombre des colonies de micro-organismes trouvées par unité de surface et on le recalcule sur l'échantillon.

9. Procédé suivant la revendication 8, caractérisé en ce que le comptage des colonies de types différents est effectué de manière séparée.

10. Dispositif pour la mise en oeuvre du procédé suivant l'une des revendications précédentes, caractérisé par le couplage ou l'intégration
a) d'une unité d'inoculation destinée à appliquer les échantillons sur un support de culture,
b) d'une unité d'incubateur pour l'incubation des cultures,
c) d'une unité de transfert pour le transfert d'une manière fidèle quant à la localisation des microcolonies formées à partir du support de culture sur un support optique sous la forme d'un poinçon dont la surface de poinçon est constituée d'une matière transparente ou réflectrice dans la gamme d'ondes souhaitée,
d) d'une unité pour localiser des colonies de microorganismes au moyen d'un microscope,
e) d'un microscope aux IR, et
f) d'une unité pour commander de manière contrôlée le fonctionnement individuel de l'appareil.

11. Dispositif suivant la revendication 10, caractérisé en outre par une unité de transmission pour passer le support optique sur l'objectif d'un microscope, une unité mécanique pour le balayage de l'échantillon transmis par utilisation du mode optique d'un microscope aux IR ou d'une unité de détection destinée à saisir des empreintes de microcolonies sur le support optique dans le mode optique et spectroscopique infrarouge d'un microscope aux IR.

12. Dispositif suivant l'une des revendications 10 et 11, caractérisé en ce que l'unité de commande de l'appareil est, parallèlement à la détection ou comme alternative à celle-ci, équipée pour la commande d'une identification.
